# FASCICULE DE BREVET EUROPEEN

(11) **EP 1 096 258 B1**
(45) Date de publication et mention de la délivrance du brevet: **25.04.2007**
(21) Numéro de dépôt: 00403010.2
(22) Date de dépôt: 30.10.2000
(51) Int. Cl.: G01N 33/68

(54) **Dosage de protéines telles que les immunoglobulines de type E (IgE) dans des sécrétions nasales**
Bestimmung von Proteinen wie Immunglobuline E (IgE) in Nasensekreten
Assay of proteins such as type E immunoglobulins (IgE) in nasal secretions

(30) Priorité: 29.10.1999 FR 9913568
(43) Date de publication de la demande: 02.05.2001
(73) Titulaire: Bloch-Michel, Etienne, 75007 Paris (FR); De Luca, Hélène, 29000 Quimper (FR)
(72) Inventeur: Bloch-Michel, Etienne, 75007 Paris (FR); De Luca, Hélène, 29000 Quimper (FR)
(74) Mandataire: Becker, Philippe

(56) Documents cités:
- FR-A- 2 670 292
- PENG Z; LEE H-B; PROUD D; NACLERIO R; ADKINSON N F JR: "RAGWEED IGE AND IGG-4 ANTIBODY IN NASAL SECRETIONS DURING IMMUNOTHERAPY" CLINICAL AND EXPERIMENTAL ALLERGY, vol. 20, 1990, pages 571-580, XP000933908
- DATABASE BIOSIS [en ligne] BIOSCIENCES INFORMATION SERVICE, PHILADELPHIA, PA, US; Accession Number PREV198681019760, 1986 NACLERIO R M; PLAUT M; ADKINSON N F; NORMAN P S JR: "EFFECT OF SEASONAL RAGWEED EXPOSURE ON IMMUNOGLOBULIN E ANTIRAGWEED ANTIBODIES IN CULTURES OF PERIPHERAL MONONUCLEAR CELLS PLASMA AND NASAL SECRETIONS" XP002144803 & INTERNATIONAL ARCHIVES OF ALLERGY AND APPLIED IMMUNOLOGY, vol. 78, 1985, pages 225-232,
- MERRETT T G; HOURI M; MAYER A L R; MERRETT J: "MEASUREMENT OF SPECIFIC IMMUNO GLOBULIN E ANTIBODIES IN NASAL SECRETION EVIDENCE FOR LOCAL PRODUCTION" CLINICAL ALLERGY, vol. 6, 1976, pages 69-73, XP000933923
- DATABASE BIOSIS [en ligne] BIOSCIENCES INFORMATION SERVICE, PHILADELPHIA, PA, US; Accession Number PREV198477072508, 1984 ITO Y: "STUDY OF IMMUNO GLOBULIN G ANTIBODY IN JAPANESE CEDAR POLLENOSIS PATIENTS 2. THE EFFECT OF IMMUNO THERAPY AND SEASONAL EXPOSURE TO CEDAR POLLEN ON SPECIFIC IMMUNO GLOBULIN G AND IMMUNO GLOBULIN E ANTIBODIES IN SERUM AND NASAL SECRETIONS" XP002144804 & JAPANESE JOURNAL OF ALLERGOLOGY, vol. 32, no. 9, 1983, pages 985-995,
- MOGI G. ET AL: 'IgE studies on respiratory tract allergies.' ARCH. OTOLARYNGOL. vol. 103, Mai 1977, pages 251 - 257, XP009032738
- LORIN M.I. ET AL: 'Quantitative composition of nasal secretions in normal subjects.' J. LAB. CLIN. MED. vol. 80, no. 2, Août 1972, pages 275 - 281

## Description

La présente invention se rapporte aux domaines techniques de la médecine, de la biologie et de la biochimie. Ses applications concernent les domaines de la santé humaine ou animale. Plus particulièrement, l'invention décrit de nouvelles méthodes qui permettent de déterminer la présence et/ou d'évaluer la quantité de protéines ou glycoprotéines par dosage dans les sécrétions nasales. L'invention décrit plus spécifiquement des méthodes particulièrement utiles pour doser la présence d'immunoglobulines, notamment d'immunoglobulines de type E (IgE) dans des sécrétions nasales. La présente invention décrit également des matériels et kits pour la mise en oeuvre de ces méthodes, ainsi que leurs applications, par exemple pour dépister ou caractériser le potentiel allergique de sujets.

Il est connu de réaliser un dosage de protéines ou glycoprotéines, telles que des IgE, à partir de sécrétions lacrymales. Ainsi, la demande de brevet française n° FR9015200 décrit un dosage quantitatif de protéines dans les larmes, au moyen d'une bandelette sur laquelle les IgE s'adsorbent, suivi d'une révélation immunologique des IgE présentes. Cette méthode, bien qu'efficace, présente toutefois certaines limitations. Il est en effet connu que l'oeil est un organe relativement isolé sur le plan immunologique, de sorte que les IgE dosées dans les larmes reflètent essentiellement une sensibilité locale (i.e., la présence éventuelle d'allergies oculaires), mais sont peu indicatives sur l'état plus général d'un sujet ou sur d'autres sensibilisations allergiques locales.

Les autres méthodes disponibles actuellement pour doser les immunoglobulines, telles que les IgE, sont essentiellement basées sur la mesure des taux d'IgE dans le sang ou le sérum. Ces techniques impliquent donc un prélèvement sanguin chez le sujet. D'autre part, dans la mesure où la sensibilité d'un sujet est dépendante non seulement des IgE circulantes, mais également des IgE fixées sur les tissus et/ou sur des cellules, les dosages sériques ne permettent pas toujours de renseigner de manière fiable ou complète sur l'état allergique d'un sujet. En particulier, ces dosages, à eux seuls, ne permettent pas de rendre compte de l'ensemble des phénomènes de développement allergiques chez un sujet.

Il existe donc un réel besoin dans l'art antérieur pour des méthodes complémentaires, simples et efficaces, permettant de doser des protéines telles que des IgE.
La détection des IgE dans les sécrétions nasales s'est révélée jusqu'ici difficile à réaliser en raison des variations spontanées du flux desdites sécrétions et de la complexité des méthodes de prélèvement (aspiration, lavage, frottis et biopsie).
La présente invention apporte une solution à ces problèmes. L'invention décrit en effet de nouvelles méthodes et compositions pour le dosage de protéines et glycoprotéines. Ces méthodes reposent principalement sur le matériel biologique utilisé pour doser les protéines, c'est-à-dire les sécrétions nasales. La présente invention montre en effet qu'il est possible de détecter, dans les sécrétions nasales, la présence de protéines ou glycoprotéines, notamment d'immunoglobulines, en particulier de type E, et que ces protéines peuvent être dosées de manière simple et efficace. La présente invention montre en outre que ce dosage, appliqué à des IgE, est particulièrement sensible, et permet de renseigner de manière fiable (et complémentaire des techniques existantes) sur l'état allergique d'un sujet. Notamment, les compositions et méthodes de l'invention permettent avantageusement de dépister les terrains allergiques chez un sujet, qu'il s'agisse d'allergies locales (par exemple rhino-pharyngées) ou générales.

Un premier objet de l'invention réside dans un procédé de détection d'IgE comprenant :
- la mise en contact d'une sécrétion nasale avec un matériel permettant l'adsorption des IgE, et
- la détection, à partir dudit matériel, de la présence des IgE par une réaction de type immunologique.

Un autre objet de l'invention réside dans un procédé de dosage d'IgE comprenant:
- la mise en contact d'une sécrétion nasale avec un matériel permettant l'adsorption des IgE et
- le dosage des IgE adsorbées sur le matériel par une réaction de type immunologique.

Ce mode de réalisation comprend avantageusement une étape d'évaluation de la quantité (ou concentration) de la protéine ou glycoprotéine concernée. A cet égard, l'étape de dosage ci-dessus comprend plus préférentiellement la détermination de la quantité de la protéine ou glycoprotéine concernée qui est adsorbée sur ledit matériel, la quantité mesurée étant rapportée à une courbe étalon établie parallèlement ou de manière préalable. Ce calcul permet de déterminer la concentration réelle de la protéine ou glycoprotéine concernée dans la sécrétion nasale. La courbe étalon est préférentiellement une courbe établie à partir de solutions de référence contenant la protéine ou glycoprotéine concernée à des concentrations définies, dosées selon le même protocole.

La présente invention décrit et concerne ainsi toute méthode qualitative ou quantitative de détection ou de dosage de molécules biologiques à partir de sécrétions nasales.

Dans le cadre de la présente invention, le terme sécrétion nasale désigne toute substance produite au niveau nasal ou naso-sinusien (comme le mucus par exemple), en particulier toute substance libérée ou sécrétée par les muqueuses nasales ou naso-sinusiennes. Il peut s'agir principalement d'écoulements observés lors de rhumes, éternuements, états allergiques ou de type allergiques, mucus, hydrorrhée, ou de toute autre substance ou sécrétion libérée en état physiologique normal ou pathologique, tel qu'on le rencontre dans les états allergiques. Le terme sécrétion nasale désigne plus préférentiellement le mucus.

L'étude des sécrétions nasales a fait l'objet de quelques travaux multidirectionnels s'intéressant soit à la biochimie (pH ou composition biochimique du mucus), soit aux facteurs cellulaires (éosinophilie locale) soit à des tests dynamiques (tests de provocation). L'invention montre à présent que des substances biologiques de type immunoglobuline peuvent être détectées ou dosées directement à partir du mucus ou autres sécrétions nasales ou naso-sinusiennes, de manière simple, sensible et reproductible.

Pour la mise en oeuvre du procédé de l'invention, la mise en contact du matériel avec les sécrétions nasales peut être réalisée soit en plaçant directement le matériel adsorbant au contact des muqueuses nasales, soit en récoltant les sécrétions nasales, et en effectuant le contact dans un dispositif approprié (lame, tube, boite, coupelle, etc). De manière préférée, la mise en contact du matériel avec les sécrétions nasales est réalisée en plaçant directement le matériel au contact des muqueuses nasales du sujet. Ceci est réalisé typiquement en introduisant manuellement le matériel adsorbant (ou une partie de celui-ci) dans une narine du sujet.

Le contact entre la sécrétion nasale et le matériel est préférentiellement réalisé pendant une période suffisante pour permettre l'adsorption de protéines ou glycoprotéines sur le matériel. Lorsqu'un dosage de type quantitatif est réalisé, le contact est préférentiellement maintenu jusqu'à ce qu'un équilibre soit atteint entre la quantité de protéines ou glycoprotéines adsorbée sur le matériel et leur concentration dans les sécrétions nasales du sujet. Généralement, ce contact peut être maintenu pendant une période allant de quelques secondes à plusieurs minutes, typiquement de 1 à 5 minutes, par exemple de 1 à 3 minutes. Il est entendu que cette période peut être ajustée aisément par le manipulateur en fonction des conditions générales, locales, du type de protéine ou glycoprotéine recherché et du but poursuivi (détection, dosage, etc).

La matériel utilisé dans le cadre de la présente invention pour adsorber les protéines et/ou glycoprotéines peut être tout support, de préférence mince, pouvant être mis au contact des sécrétions nasales, notamment introduit dans la narine sans l'agresser. Ce matériel peut avoir des formes différentes, comme par exemple être filiforme, en forme de pastille ou de disque, d'éponge chirurgicale, d'embout, de bâtonnet, de bandelette, etc. Le matériel permettant l'adsorption comprend préférentiellement au moins une partie composée d'une matière adsorbante, de préférence non agressive ou toxique pour le sujet, lorsque la mise en contact est réalisée directement sur les muqueuses nasales. Il peut s'agir notamment d'une matière cellulosique (par exemple de faible densité), de polymères, de complexes macromoléculaires, d'origine naturelle ou synthétique, seuls ou en combinaisons. Il s'agit de préférence d'une matière cellulosique.

Dans un mode préféré de mise en oeuvre de l'invention, et en particulier pour l'évaluation des IgE, le matériel adsorbant est constitué de bandelettes comprenant au moins une partie en papier cellulosique de faible densité, à fibres couchées ou non. A titre d'exemples particuliers, on peut citer notamment les bandelettes pour test de Schirmer des Laboratoires Faure (Annonay, France) (papier type Whatman n° 1) ou des bandelettes réalisées avec du papier référence n° 2312 des Laboratoires Schleicher & Schuell (Dassel, Allemagne). Les références n° 566 et n° 1573 de même provenance conviennent également. Malgré une moins bonne sensibilité, on peut encore citer, du même fournisseur, les références n° 1577, 1575, 1506, 1507, 903, 589/2, 589/3, 589/5, 589/6, 572 et 512.

Les bandelettes peuvent avantageusement avoir des dimensions de l'ordre de 55 mm x 5 mm. Lors du prélèvement, on laisse imbiber par exemple les 15 premiers millimètres environ de la bandelette, quelques minutes suffisant à cette opération.

Les bandelettes peuvent, ou non, être prévues pour une séparation de l'extrémité mise au contact des muqueuses d'avec le reste de la bandelette. Mais, en fait, on peut, après prélèvement, garder la bandelette entière, y compris pendant les étapes ultérieures. La bandelette peut ensuite être conservée, après le prélèvement, pendant plusieurs semaines avant l'étape de révélation ou de dosage.

L'étape de détection ou dosage peut être réalisée par toute technique immuno-chimique connue de l'homme du métier, basée sur l'utilisation d'anticorps et/ou d'antigènes spécifiques, seuls ou en combinaisons, pouvant être marqués et révélés selon différentes stratégies (marquages colorimétriques, enzymatiques, fluorescents, radioactifs, de type avidine-biotine, etc.).

En particulier, l'étape immunologique de détection ou dosage peut mettre en oeuvre des anticorps dirigés contre la protéine/glycoprotéine concernée pour capter celle-ci, puis assurer la détection, ou la détermination quantitative, des complexes antigène-anticorps ainsi formés. Cette méthode est de préférence une méthode "sandwich" du type décrit dans l'article de E. BLOCH-MICHEL et L. HELLEBOID, Revue française des Laboratoires n° 207, mai 1990, pages 43 à 46. Selon ce mode de réalisation, le matériel adsorbant (ou la partie de matériel adsorbant ayant été au contact des sécrétions nasales) est placé dans une solution, contenue dans un tube ou analogue (plaque, boîte, flasque, ampoule, etc.), comprenant deux séries d'anticorps dirigés contre la protéine/glycoprotéine concernée : des anticorps marqués libres d'une part, et des anticorps en phase solide (généralement non marqués), d'autre part. Les protéines passent dans la solution et se fixent sur les anticorps marqués. Chaque complexe ainsi obtenu se trouve fixé sur un anticorps en phase solide. La présence ou la quantité de protéine ou glycoprotéine concernée peut alors être déterminée par détection ou mesure de l'intensité du marquage fixé dans le tube.

Les anticorps peuvent être mono- ou polyclonaux. Les anticorps en phase solide peuvent être des anticorps immobilisés sur une phase solide telle que notamment disque de papier, bille, etc., ou directement immobilisés sur la surface interne du tube en matière plastique (anticorps fixés à la paroi intérieure) servant de récipient pour l'ensemble de la réaction, etc. La fixation des anticorps sur le support solide peut être réalisée par toute technique connue de l'homme du métier (par adsorption, fixation covalente, etc.).

La méthode immuno-chimique peut aussi mettre en oeuvre, dans les conditions décrites plus haut pour les anticorps, des réactions d'antigènes, tels que des allergènes, avec les anticorps, notamment dans une réaction de type "sandwich" telle que citée ci-dessus, auquel cas on utilise avantageusement un anticorps et un antigène, l'un étant marqué et libre, l'autre immobilisé en phase solide.

En plus de ces réactions de type "sandwich", la présente invention peut également utiliser toute autre méthode de détection ou de dosage quantitatif d'antigènes dans une solution. A cet égard, on peut citer notamment différentes techniques immuno-enzymatiques (de type ELISA, etc.) ou radio-immunologiques (de type RIA, etc.).

De préférence, au cours de l'étape de détection ou de dosage, les tubes (ou autres containers) de réaction sont agités afin d'augmenter la sensibilité, c'est-à-dire de permettre la mesure de très faibles quantités.

Comme indiqué ci-avant, pour un dosage quantitatif de protéines ou glycoprotéines, les résultats obtenus sont généralement rapportés à une courbe étalon, établie à partir de solutions de référence dosées selon le même protocole. Ces solutions de référence peuvent être des solutions produites artificiellement, par dilution (ou dilutions sérielles) de la protéine ou glycoprotéine pure. Il peut également s'agir d'échantillons biologiques comme par exemple un échantillon de larme ou de sang (ou de sérum). A cet égard, dans un mode particulier de mise en oeuvre, le procédé de l'invention comprend une étape supplémentaire de comparaison des quantités mesurées dans les sécrétions nasales avec les quantités mesurées dans un autre échantillon biologique, tel que les larmes, le sang ou le sérum.

Les procédés de l'invention peuvent être utilisés pour détecter ou doser différents types de protéines ou glycoprotéines. Il peut s'agir notamment d'immunoglobulines (IgG, IgM, IgE, etc.), de protéines inflammatoires, ou d'autres protéines ou glycoprotéines impliquées dans des phénomènes physiologiques ou physiopathologiques, ou de marqueurs utilisables pour la réalisation de dosages diagnostiques. Préférentiellement, il s'agit d'immunoglobulines, encore plus préférentiellement d'immunoglobulines de type E (IgE). Les résultats présentés dans les exemples montrent en effet que les IgE peuvent être détectées dans les sécrétions nasales, et que les dosages de l'invention offrent une grande sensibilité, permettant de détecter des variations de quantité d'IgE corrélées avec un état allergique local ou général.

A cet égard, un objet particulier de l'invention réside dans une méthode de dépistage, de suivi ou de caractérisation d'une allergie chez un sujet, caractérisée en ce qu'elle comprend une étape de détection et/ou de dosage des IgE présentes dans les sécrétions nasales.

Cette détection ou ce dosage sont avantageusement réalisés dans les conditions décrites ci-avant.

Le dépistage indique que le caractère allergique du sujet considéré n'est pas encore connu ou révélé, ou est à un stade précoce non encore confirmé. La méthode de l'invention est également applicable au suivi et/ou à la caractérisation d'une allergie, c'est-à-dire à une étude de l'évolution du stade allergique d'un sujet, ou à la détermination du type d'allergie présentée, notamment son caractère local ou général.

Le procédé de l'invention peut être utilisé pour doser simultanément plusieurs protéines ou glycoprotéines d'intérêt, ou plusieurs types de protéines ou glycoprotéines d'intérêt. Il peut s'agir notamment des IgE totales, ou bien de certaines igE spécifiques d'antigènes (allergènes) déterminés. Selon une application préférée, le procédé de l'invention comprend une détection ou un dosage des IgE présentes dans les sécrétions nasales, sans distinction de spécificité antigénique (IgE totales).

Le procédé peut donc non seulement s'appliquer au dosage des IgE nasales totales, mais aussi au dosage des IgE spécifiques, notamment à des concentrations faibles. A fortiori, on peut procéder à un dosage quantitatif des IgE spécifiques.

Pour les IgE spécifiques, on utilise avantageusement des allergènes ou réactogènes qui peuvent être insolubilisés, de la façon décrite pour les anticorps, ou non, ou marqués ou non.

Par ailleurs, le procédé selon l'invention peut permettre d'établir non seulement des diagnostics dans les cas d'allergies locales (de type rhinopharyngées), mais aussi dans le cas des allergies générales. Dans ce dernier cas, la sensibilité accrue du procédé permet la quantification des IgE transsudées. A cet égard, le procédé de l'invention peut notamment être utilisé pour dépister ou suivre l'évolution d'allergies telles que asthme, eczéma, rhinites, sinusites allergiques, etc.

Comme indiqué ci-avant, l'invention est également applicable au dosage nasal de substances reflétant un état inflammatoire ou pathologique et notamment des substances susceptibles de se trouver dans les sécrétions nasales dans des plages de concentrations faibles et de préférence assez peu étendues.

Les matériels et procédés de l'invention peuvent être utilisés pour détecter ou doser des protéines ou glycoprotéines dans les sécrétions nasales de tout sujet humain, adulte ou enfant, sain ou présentant une pathologie. En outre, l'invention peut également être appliquée à la détection ou au dosage de protéines ou glycoprotéines dans les sécrétions nasales de tout autre mammifère (notamment canins, bovins, ovins, équins, porcins, etc).

La présente invention est également relative à des kits pour la mise en oeuvre des méthodes décrites ci-avant. Ces kits comprennent avantageusement des solutions permettant la réalisation d'une réaction immunologique, et notamment au moins un anticorps anti-IgE. Cet anticorps peut être un polyclonal ou un monoclonal, il peut être marqué ou non. Les kits de l'invention comprennent plus préférentiellement un dispositif pour réaliser la réaction immunologique, tel que par exemple un tube, puits, boite ou flacon. Par ailleurs, les kits de l'invention peuvent en outre comprendre un matériel adsorbant, utilisable pour réaliser le prélèvement.

Dans un mode particulier, les kits selon l'invention comprennent un tube (ou analogue) recouvert d'anticorps anti-IgE et une solution d'anticorps anti-IgE couplé à une enzyme (par exemple la peroxydase). Plus préférentiellement, ils comprennent en outre un matériel adsorbant (notamment une bandelette) imprégnée d'IgE étalon et/ou une solution de lavage et/ou un substrat chromogène de l'enzyme (par exemple l'OPD). Ces kits peuvent être utilisés aisément pour détecter ou doser les IgE à partir de sécrétions nasales, selon la présente invention.

Un autre objet de l'invention réside également dans l'utilisation d'un matériel permettant l'adsorption de protéines ou glycoprotéines pour la détection ou le dosage de protéines ou glycoprotéines à partir d'une sécrétion nasale d'un sujet, par mise en contact directe dudit matériel avec une muqueuse nasale dudit sujet.
Le matériel est préférentiellement tel que défini ci-avant, et est utilisé pour détecter la présence ou pour doser les immunoglobulines.

Un autre objet de l'invention réside également dans l'utilisation d'un matériel permettant l'adsorption de protéines ou glycoprotéines pour la fabrication d'un produit ou kit pour la mise en oeuvre d'une méthode de diagnostique, dépistage, suivi ou caractérisation d'une allergie chez un sujet.

D'autres aspects et avantages de la présente invention apparaîtront à la lecture des exemples qui suivent, qui doivent être considérés comme illustratifs et non limitatifs. Ces exemples illustrent notamment le principe du dosage des IgE totales par une technique immuno-enzymatique dans des sécrétions nasales prélevées à l'aide d'une bandelette de papier.

### 1. Principe du dosage

La détection immuno-enzymatique a été effectuée par une technique de type « sandwich » utilisant deux anticorps monoclonaux anti-IgE. La réaction se déroule en deux étapes :
- Une réaction immunologique : les IgE à doser (ou détecter) sont incubées 3h à 18-25°C dans des tubes en présence de deux anticorps monoclonaux anti-IgE dont l'un est fixé à la paroi des tubes et l'autre, en phase liquide, est marqué à la peroxydase. L'incubation est suivie d'une phase de lavage des tubes.
- Une révélation enzymatique : on ajoute dans les tubes un substrat chromogène de type OPD (orthophénylènediamine). Après incubation 30 minutes à 18-25°C, on ajoute un agent bloquant (acide sulfurique) et on lit la coloration obtenue au spectro-photomètre.

Au cours de la même série de dosage, on effectue un blanc réactif (tube sans IgE), des étalons (tubes contenant des quantités connues d'IgE) et un contrôle positif.

### 2. Description des réactifs (Kit de dosage)

Les réactifs suivants sont utilisés dans la mise en oeuvre de la méthode :
- Bandelettes de papier pour effectuer le prélèvement nasal,
- Tubes en polystyrène recouverts d'anticorps anti-IgE
- Bandelettes imprégnées d'IgE étalon
- Bandelettes imprégnées de contrôle positif
- Solution d'anticorps anti-lgE marqué à la peroxydase
- Solution de lavage
- Chromogène (OPD en comprimés avec diluant)
- Agent bloquant (acide sulfurique)

### 3. Mode Opératoire

Le procédé est réalisé comme suit.

Pour la réaction immunologique, les bandelettes préalablement mises en contact avec les sécrétions nasales (ou les bandelettes étalon ou contrôle) sont placées dans les tubes (sauf le tube blanc réactif). 300 µl d'anticorps conjugué à la peroxydase sont ajoutés et les tubes sont maintenus sous agitation pendant 3 heures à température ambiante, à une vitesse de 360 rpm. Le milieu réactionnel de tous les tubes est aspiré, et la solution de lavage est répartie dans les tubes (2 ml par tube) et les bandelettes sont retirées des tubes. La solution est aspirée à nouveau, et l'opération de lavage est renouvelée deux fois. Les solutions sont ensuite aspirées soigneusement.

Pour la révélation enzymatique, 300 µl de solution de révélation sont répartis dans chaque tube. Après incubation pendant 30 minutes à température ambiante et à l'abri de la lumière, 1 ml d'agent bloquant est introduit dans chaque tube. Les tubes sont agités (par exemple sur agitateur de type Vortex). La coloration obtenue est lue à 492 nm contre le blanc réactif.

### 4. Résultats

Différents essais préliminaires ont été réalisés chez des sujets humains, en condition pathologique ou saine, afin de doser les IgE présentes dans leurs sécrétions nasales (y compris naso-sinusiennes).

### Les résultats obtenus peuvent se résumer comme suit:

Dans une première série d'expériences, le test des IgE nasales de l'invention a été comparé, chez une cinquantaine de sujets, aux tests de provocation nasale (thermique et éosinophile). Les résultats obtenus montrent que le test des IgE nasales de l'invention est plus sensible que le test de provocation éosinophile, et plus sélectif que le test de provocation thermique.

Dans une deuxième série d'expériences, le test des IgE nasales de l'invention a été comparé au test cutané, chez 17 sujets humains ayant consulté pour des manifestations rhinopharyngées évoquant l'allergie. Chez 10 patients, alors que le test cutané était négatif, le test de l'invention a permis de mettre en évidence une élévation importante des IgE dans les sécrétions nasales. Ces expériences semblent indiquer que les tests de l'invention permettent une détection plus fiable que les tests cutanés antérieurs. Ces résultats semblent également confirmer la prédominance de l'hypersensibilité locale sur l'hypersensibilité générale.

Les résultats obtenus montrent en outre que les IgE recueillies sur les bandelettes peuvent être dosées directement et de façon proportionnelle à leur concentration dans le milieu à doser, sans qu'il soit nécessaire de calculer le poids ou la quantité recueillie. Ce phénomène tient au fait qu'il s'établit très rapidement (en quelques minutes) un équilibre entre les IgE qui s'adsorbent sur le papier et celles qui restent dans la solution. Ensuite, lorsque le papier est incubé avec la solution d'anticorps anti-lgE, les IgE quittent le papier pour former un complexe antigène-anticorps qui peut alors être dosé.

Le dosage selon l'invention est avantageux puisqu'il peut être réalisé en une seule étape, et qu'il permet, par agitation des tubes, la détection de très faibles quantités de protéines (inférieures à 2 kUl/l).

Dans une troisième série d'expériences, le test des IgE nasales de l'invention est pratiqué sur un groupe témoin incluant 9 sujets humains (5 femmes et 4 hommes) âgés de 25 à 55 ans et sur un groupe de 17 patients (7 femmes et 10 hommes) de 7 à 61 ans.
Le groupe témoin est composé de sujets ne présentant ni symptôme de rhinite ni symptôme d'allergie et n'ayant aucun antécédent personnel ou familial. Le second groupe est composé de sujets ayant consulté pour des symptômes de rhinite chronique présents sur une période supérieure à trois mois.
Les bandelettes ont été mises au contact des muqueuses nasales des sujets pendant 60 secondes, puis envoyées au laboratoire (sous 15 jours) pour l'analyse dans les conditions décrites ci-avant.
Les résultats obtenus montrent une densité optique moyenne dans les sécrétions nasales des sujets contrôlés de 0,136, et une densité optique moyenne dans les sécrétions nasales des sujets symptomatiques de 1,752.
Le test des IgE nasales pratiqué selon l'invention a ainsi permis de mettre en évidence une différence significative dans la sécrétion locale d'IgE entre le groupe témoin et le groupe de patients et donc de clairement distinguer les sujets allergiques des sujets sains.

## Revendications

1. Procédé de détection ou de dosage d'IgE, comprenant:
- la mise en contact d'une sécrétion nasale avec un matériel permettant l'adsorption des IgE comprenant au moins une partie composée d'une matière adsorbante choisie parmi une matière cellulosique, un polymère ou un complexe macromoléculaire, d'origine naturelle ou synthétique, seuls ou en combinaisons, en plaçant directement le matériel au contact des muqueuses nasales d'un sujet, et
- la détection ou le dosage, à partir dudit matériel, de la présence d'IgE par une réaction de type immunologique, cette étape de détection ou de dosage comprenant l'incubation du matériel adsorbant, ou la partie de matériel adsorbant ayant été au contact des sécrétions nasales, dans une solution comprenant deux séries d'anticorps dirigés contre les IgE : des anticorps marqués libres d'une part, et des anticorps en phase solide d'autre part.

2. Procédé selon la revendication 1, **caractérisé en ce que** l'étape de dosage comprend la détermination de la quantité d'IgE concernée qui est adsorbée sur ledit matériel, la quantité mesurée étant rapportée à une courbe étalon.

3. Procédé selon l'une quelconque des revendications précédentes, **caractérisé en ce que** le matériel adsorbant comprend au moins une partie composée d'une matière adsorbante cellulosique non agressive ou toxique pour le sujet.

4. Procédé selon l'une quelconque des revendications précédentes, **caractérisé en ce que** le matériel adsorbant est constitué d'un support, filiforme, en forme de pastille ou de disque, d'éponge chirurgicale, d'embout, de bâtonnet ou de bandelette.

5. Procédé selon l'une quelconque des revendications précédentes, **caractérisé en ce que** les anticorps sont marqués par un marquage colorimétrique, enzymatique, fluorescent, radioactif et/ou de type avidine-biotine.

6. Procédé selon l'une des revendications précédentes, **caractérisé en ce que** l'étape de détection ou de dosage est réalisée par méthode immunologique ELISA.

7. Procédé selon l'une des revendications précédentes, **caractérisé en ce que** les anticorps sont des anticorps monoclonaux ou polyclonaux.

8. Procédé selon l'une des revendications précédentes, pour la détection ou le dosage des IgE totales.

## Claims

1. A method for detecting or dosing IgE, comprising :
- contacting a nasal secretion with a material allowing IgE adsorption comprising at least a part consisting of an adsorbing material selected from a material made of cellulose, a polymer or a macromolecular complex, of natural or synthetic origin, alone or in admixtures, by directly contacting the material with nasal mucosa of a subject, and
- detecting or dosing, from said material, the presence of IgE by a reaction of an immunological type, this detecting or dosing step comprising incubating the adsorbing material, or the part of the adsorbing material that has been contacted with nasal secretions, in a solution comprising two series of antibodies directed against the IgE: free labelled antibodies on the one hand and antibodies in solid phase on the other hand.

2. The method according to claim 1, **characterized in that** the dosing step comprises determining the concerned IgE quantity that is adsorbed on said material, the measured quantity being compared to a measurement standard.

3. The method according to anyone of the above claims, **characterized in that** the adsorbing material comprises at least a part made of a cellulosic adsorbing material which is non aggressive or non toxic for the subject.

4. The method according to anyone of the above claims, **characterized in that** the adsorbing material is made of a filiform support, with a chip or disc, chirurgical sponge, adapter, stick or band shape.

5. The method according to anyone of the above claims, **characterized in that** the antibodies are labelled with a colorimetric, an enzymatic, a fluorescent, a radioactive and/or an avidin-biotin type label.

6. The method according to one of the above claims, **characterized in that** the detecting or dosing step is performed using immunological ELISA method.

7. The method according to one of the above claims, **characterized in that** the antibodies are monoclonal or polyclonal antibodies.

8. The method according to one of the above claims, for detecting or dosing total IgE.

## Patentansprüche

1. Verfahren zum Nachweis oder zur quantitativen Bestimmung von IgE, umfassend:
- das Inkontaktbringen eines Nasensekrets mit einem Material, das die Adsorption der IgE erlaubt und wenigstens einen Teil umfasst, der aus einem Adsorptionsmittel zusammengesetzt ist, das aus einem Cellulose-Material, einem Polymer oder einem Makromolekülkomplex, natürlichen oder synthetischen Ursprungs, allein oder in Kombinationen, ausgewählt ist, wobei das Material in direkten Kontakt mit den Nasenschleimhäuten einer Person gebracht wird, und
- den Nachweis oder die quantitative Bestimmung des Vorhandenseins von IgE aus besagtem Material durch eine Reaktion immunologischer Art, wobei dieser Schritt des Nachweises oder der quantitativen Bestimmung die Inkubation des Adsorptionsmaterials oder des Teils des Adsorptionsmaterials, das mit den Nasensekreten in Kontakt gewesen ist, in einer Lösung umfasst, die zwei gegen die IgE gerichtete Antikörpertypen enthält: einerseits freie markierte Antikörper und andererseits Antikörper an einer festen Phase.

2. Verfahren nach Anspruch 1, **dadurch gekennzeichnet, dass** der Schritt der quantitativen Bestimmung die Bestimmung der betreffenden Menge an IgE umfasst, die an dem besagten Material adsorbiert ist, wobei die gemessene Menge auf eine Eichkurve bezogen ist.

3. Verfahren nach einem beliebigen der vorhergehenden Ansprüche, **dadurch gekennzeichnet, dass** das Adsorptionsmaterial wenigstens einen Teil umfasst, der aus einem für die Person nicht belastenden oder toxischen Cellulose-Adsorptionsmittel zusammengesetzt ist.

4. Verfahren nach einem beliebigen der vorhergehenden Ansprüche, **dadurch gekennzeichnet, dass** das Adsorptionsmaterial aus einem Träger in Form eines Fadens, Pastille oder Scheibe, chirurgischen Schwamms, Spitze, Stäbchens oder Streifens besteht.

5. Verfahren nach einem beliebigen der vorhergehenden Ansprüche, **dadurch gekennzeichnet, dass** die Antikörper mit einer kolorimetrischen, enzymatischen, fluoreszierenden, radioaktiven Markierung und/oder einer Markierung des Typs Avidin-Biotin markiert sind.

6. Verfahren nach einem der vorhergehenden Ansprüche, **dadurch gekennzeichnet, dass** der Schritt des Nachweises oder der quantitativen Bestimmung mit einem immunologischen ELISA-Verfahren durchgeführt wird.

7. Verfahren nach einem der vorhergehenden Ansprüche, **dadurch gekennzeichnet, dass** die Antikörper monoklonale oder polyklonale Antikörper sind.

8. Verfahren nach einem der vorhergehenden Ansprüche zum Nachweis oder zur quantitativen Bestimmung von Gesamt-IgE.
